# EUROPÄISCHE PATENTANMELDUNG

(11) **EP 1 584 293 A1**
(43) Veröffentlichungstag der Anmeldung: **12.10.2005**
(21) Anmeldenummer: 05005338.8
(22) Anmeldetag: 11.03.2005
(51) Int. Cl.: A61B 17/02, A61B 17/00, A61F 5/00

(54) **Laparoskopisches Instrument**

(30) Priorität: 06.04.2004 AT 6022004
(71) Anmelder: AMI Agency for Medical Innovations GmbH, 6800 Feldkirch (AT)
(72) Erfinder: Erhard, Martin, 6780 Silbertal (AT)
(74) Vertreter: Hefel, Herbert

(57) **Zusammenfassung**

Ein laparoskopisches Instrument umfasst einen Handgriff (1), einen vom Handgriff (1) getragenen Schaft (2) und einen am distalen Ende des Schafts (2) angeordneten Arbeitskopf (3), wobei der Schaft (2) einen distalen, krümmbaren Abschnitt (4) aufweist und zur Verstellung der Krümmung des krümmbaren Abschnitts (4) ein von einem Draht oder Seil gebildetes Zugelement (7) vom Bereich des Handgriffs (1) bis über den krümmbaren Abschnitt (4) verläuft und auf der distalen Seite des krümmbaren Abschnitts (4) am Schaft (2) oder am Arbeitskopf (3) befestigt ist und ein im Bereich des Handgriffs (1) angeordnetes Betätigungsorgan (5) mit dem Zugelement (7) zur Zugbeaufschlagung des Zugelements (7) zusammenwirkt und wobei über den krümmbaren Abschnitt (4) mehrere in Längsrichtung des krümmbaren Abschnitts (4) voneinander beabstandete Führungsbügel (8) für das Zugelement (7) angeordnet sind. Über den krümmbaren Abschnitt (4) erstreckt sich weiters eine im unbelasteten Zustand gerade Blattfeder (19), wobei die Abhebung des Zugelements (7) von der Blattfeder (19) durch die Führungsbügel (8) begrenzt ist.

## Beschreibung

Die Erfindung betrifft ein laparoskopisches Instrument mit einem Handgriff , einem vom Handgriff getragenen Schaft und einem am distalen Ende des Schafts angeordneten Arbeitskopf, wobei der Schaft einen distalen, krümmbaren Abschnitt aufweist und zur Verstellung der Krümmung des krümmbaren Abschnitts ein von einem Draht oder Seil gebildetes Zugelement vom Bereich des Handgriffs bis über den krümmbaren Abschnitt verläuft und auf der distalen Seite des krümmbaren Abschnitts am Schaft oder am Arbeitskopf befestigt ist und ein im Bereich des Handgriffs angeordnetes Betätigungsorgan mit dem Zugelement zur Zugbeaufschlagung des Zugelements zusammenwirkt und wobei über den krümmbaren Abschnitt mehrere in Längsrichtung des krümmbaren Abschnitts voneinander beabstandete Führungsbügel für das Zugelement angeordnet sind.

Laparoskopische Instrumente sind chirurgische Instrumente für laparoskopische Operationen. Bei solchen wird eine Zugangsöffnung in eine Körperhöhle an der für den Eingriff gewünschten Stelle geschaffen. Zur Schaffung einer solchen Zugangsöffnung werden üblicherweise Trokare eingesetzt. Solche Trokare sind hülsenförmig ausgebildet und besitzen einen Durchgangskanal, durch welchen hindurch die Operation mittels der laparoskopischen Instrumente ausgeführt wird. Diese chirurgische Technik gehört zu den minimal invasiven chirurgischen Techniken (LIS-Technik).

Bekannt ist ein laparoskopisches Instrument mit einem Handgriff, einem vom Handgriff getragenen Schaft und einem am distalen Ende des Schafts angeordneten Arbeitskopf, bei dem ein distaler krümmbarer Abschnitt des Schafts von mehreren gelenkig miteinander verbundenen Gliedern gebildet wird. Neben einem Zugelement, das von einem am Handgriff angeordneten Betätigungsorgan beaufschlagbar ist, verläuft ein zweiter Seilzug durch den krümmbaren Abschnitt, der einerseits am Handgriff, andererseits am Arbeitskopf festgelegt ist und in welchem eine Schraubenfeder angeordnet ist. Dieser zweite Seilzug dient dazu, den krümmbaren Abschnitt gerade zu richten, wenn das mit dem Betätigungsteil zusammenwirkende Zugelement nicht über dieses Betätigungsteil angezogen und verschoben wird, sondern locker gelassen wird. Die einstellbare Krümmung des krümmbaren Abschnitts dient dazu, um mit dem Arbeitskopf Strukturen im Körperinneren zu umfahren, beispielsweise Hohlorgane oder Gefäße. Mit dem Arbeitskopf kann beispielsweise in der Folge ein Implantat, ein Faden oder ein anderer Gegenstand um Hohlorgan oder ein Gefäß herumgezogen werden. Nachteilig an diesem herkömmlichen Instrument ist unter anderem dessen relativ aufwändiger Aufbau, der zu entsprechenden Kosten dieses Instruments führt. Weiters ist eine rückstandsfreie Reinigung dieses Instruments nach Durchführung einer Operation praktisch nicht möglich. Zwischen den Gliedern des krümmbaren Abschnitts und an den Zugseilen setzen sich bei der Operation Blut und Körperflüssigkeiten fest, von denen auch nach der Spülung und der Sterilisation des Instruments Rückstände zurückbleiben, welche zu einem Infektionsrisiko führen.

Bekannt ist weiters, beispielsweise aus der DE 41 33 800 C1, ein laparoskopisches Instrument, bei welchem aus dem distalen Ende eines an einem Handgriff angebrachten Rohrs eine Blattfeder herausschiebbar ist, und zwar mittels eines am Handgriff angeordneten Betätigungsorgans. Die Blattfeder ist im innerhalb des Rohrs liegenden Zustand gespannt und nimmt beim Herausschieben aus dem Rohr ihre vorgegebene Krümmung an, die sie ohne einwirkende äußere Kräfte aufweist. Das distale Ende dieser Flachfeder bildet hierbei den Arbeitskopf des Instruments. Dieser Arbeitskopf dient beispielsweise zur Dissektion von Gewebe und kann auch mit einem Schlitz versehen sein, in welchen ein Gegenstand, beispielsweise Faden,einhängbar ist, um diesen um ein Hohlorgan oder ein Gefäß herumzuziehen. Nachteilig an diesem Instrument ist u. a. die problematische Reinigung, insbesondere von in das vordere Ende des Rohrs eingedrungenen Körperflüssigkeiten. Auch bestehen Einschränkungen hinsichtlich von Änderungen des Krümmungsradius im an den Arbeitskopf anschließenden Abschnitt.

Ein Umfahrungsinstrument mit einem um einen Gegenstand herum legbaren, länglichen, streckbaren und abbiegbaren Arm, an dessen freiem Ende ein Haltewerkzeug angeordnet ist, wobei der Arm mindestens über einen Teil seiner Länge als Schlauch aus einem dehnbaren Material ausgebildet ist, ist aus der DE 43 25 969 C1 bekannt. Die Längsdehnbarkeit des Schlauchs ist auf zwei einander gegenüberliegenden Seiten verschieden und das Innere des Schlauchs ist mit einer ein fließfähiges Medium unter Druck in den Schlauch einfüllenden Druckquelle verbunden.

Ein laparoskopisches Instrument der eingangs genannten Art ist aus der US 6,645,218 B1 bekannt. Der krümmbare Abschnitt des Schafts ist elastisch ausgebildet, wobei von der Oberseite des Schafts eine Reihe von keilförmigen Schlitzen ausgehen und von der Unterseite des Schafts eine Reihe von jeweils zwischen den von der Oberseite ausgehenden Schlitzen angeordneten keilförmigen Schlitzen ausgehen. Von einem am Handgriff angeordneten Betätigungsorgan gehen zwei parallel zueinander verlaufende Zugelemente aus, die sich über den Schaft bis zu einer distal des krümmbaren Abschnitts liegenden Befestigungsstelle erstrecken. Im krümmbaren Abschnitt sind diese Zugelemente von den durch die keilförmigen Schlitze beabstandeten Abschnitten des Schafts geführt, welche somit als Führungsbügel für die Zugelemente angesehen werden können. Die laterale Steifigkeit des Schafts des Instruments in seinem krümmbaren Abschnitt ist relativ gering.

Aufgabe der Erfindung ist es, ein verbessertes laparoskopisches Instrument der eingangs genannten Art bereitzustellen. Erfindungsgemäß gelingt dies durch ein laparoskopisches Instrument mit den Merkmalen des Anspruchs 1.

Durch die erfindungsgemäße Ausbildung, bei der sich über den krümmbaren Abschnitt eine Blattfeder erstreckt, wird eine hohe Biegesteifigkeit in lateraler Richtung erzielt, d. h. in eine Richtung senkrecht zur Ebene, in welcher der Schaft in seinem gekrümmten Zustand liegt. Es kann dadurch eine sehr gute Kraftübertragung in dieser lateralen Richtung erzielt werden, wodurch beispielsweise eine stumpfe Dissektion erleichtert wird.

Durch die Erfindung wird weiters eine sehr kostengünstige Herstellung des Instruments ermöglicht. Das Instrument kann dadurch u. a. auch als Einmalinstrument ausgebildet werden, sodass Reinigungsprobleme entfallen und ein vermindertes Infektionsrisiko erreicht werden kann.

Ein erfindungsgemäßes Instrument kann weiters in einer Weise ausgebildet werden, bei der eine rückstandsfreie Reinigung ermöglicht wird. Hierbei wird der krümmbare Abschnitt vorteilhafterweise von einem äußeren Hüllschlauch umgeben, der sich vorzugsweise über die gesamte Länge des Schafts erstreckt.

In einer bevorzugten Ausführungsform der Erfindung ist ein Formteil aus Kunststoff vorhanden, welches sich über den krümmbaren Abschnitt erstreckt und welches die Führungsbügel aufweist, wobei aufeinanderfolgende Führungsbügel jeweils durch Verbindungsabschnitte des Formteils verbunden sind, die mit den freien Enden der Schenkel der U-förmigen Führungsbügel verbunden sind. Die Blattfeder verläuft hierbei zwischen den Schenkeln der Führungsbügel.

Vorzugsweise ist der krümmbare Abschnitt mit Silikon auf eine über die Länge des krümmbaren Abschnitts glatte Außenkontur ausgegossen, wobei die Ausbildung einer zylindermantelförmigen Außenkontur besonders bevorzugt ist. Es kann dadurch, insbesondere zusammen mit dem vorzugsweise eingesetzten äußeren Hüllschlauch, eine zumindest im geraden Zustand des krümmbaren Abschnitts im wesentlichen glatte Außenfläche erreicht werden.

Weitere Vorteile und Einzelheiten der Erfindung werden im Folgenden anhand der beiliegenden Zeichnung erläutert. In dieser zeigen:
- Fig. 1: einen Längsschnitt durch ein Ausführungsbeispiel eines erfindungsgemäßen laparoskopischen Instruments;
- Fig. 2: einen vergrößerten Ausschnitt von Fig. 1;
- Fig. 3: einen Querschnitt entlang der Linie A-A von Fig. 2;
- Fig. 4: einen vorderen Teil des Schaftes (ohne den Hüllschlauch und die Silikon-Vergussmasse) in einer perspektivischen Darstellung;
- Fig. 5: eine perspektivische Darstellung des Formteils;
- Fig. 6, 7 und 8: eine Ansicht von unten, Seitenansicht und eine Ansicht von oben des Formteils.

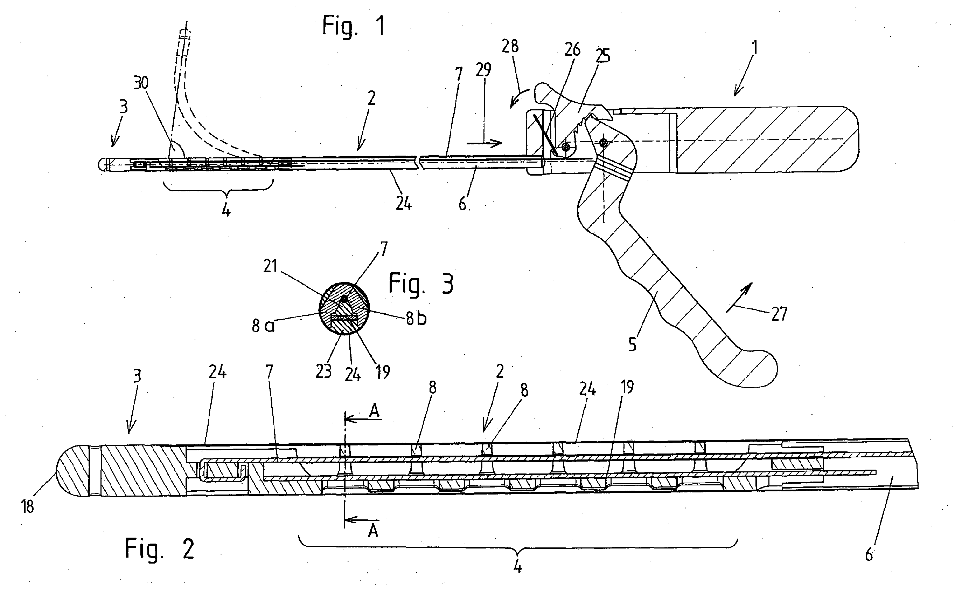

Ein Ausführungsbeispiel eines erfindungsgemäßen laparoskopischen Instruments ist in den Fig. dargestellt. Das Instrument besitzt einen Handgriff 1, einen am Handgriff befestigten Schaft 2 und einen am distalen Ende des Schaftes 2 angeordneten Arbeitskopf 3. Der Schaft 2 umfasst einen distalen, krümmbaren Abschnitt 4, der zwischen einer geraden Stellung, die in Fig. 1 in durchgehenden Linien dargestellt ist, und einer gekrümmten Stellung, die in Fig. 1 durch strichlierte Linien angedeutet ist, verstellbar ist. Der krümmbare Abschnitt 4 liegt distal der Längsmitte des Schaftes 2, vorzugsweise in einem dem Arbeitskopf 3 benachbarten Bereich, wobei der Abstand des krümmbaren Abschnitts 4 vom Arbeitskopf 3 günstigerweise weniger als die Hälfte der Länge des krümmbaren Abschnitts 4 beträgt. Zur Verstellung der Krümmung des krümmbaren Abschnitts 4 dient ein im Bereich des Handgriffs 1 angeordnetes Betätigungsorgan 5, welches im gezeigten Ausführungsbeispiel von einem am Handgriff 1 verschwenkbar gelagerten Betätigungshebel gebildet wird.

Der proximale Teil des Schafts 2 wird von einem Rohr 6 gebildet, das mit seinem proximalen Endabschnitt in eine Bohrung im Handgriff 1 ragt und in dieser befestigt ist, beispielsweise angeklebt ist. Am distalen Ende des Rohrs 6 ist der krümmbare Abschnitt 4 befestigt, beispielsweise angeklebt.

Zur Verstellung der Krümmung des krümmbaren Abschnitts 4 dient weiters ein Zugelement 7, welches von einem Draht oder einem Seil (bestehend aus mehreren Litzen) gebildet wird. Das Zugelement 7 verläuft vom Bereich des Handgriffs 1 bis über den krümmbaren Abschnitt 4 und ist auf der distalen Seite des krümmbaren Abschnitts am Schaft 2 befestigt. Am auf der Seite des Handgriffs 1 liegenden Ende ist das Zugelement 7 am Betätigungsorgan 5 befestigt.

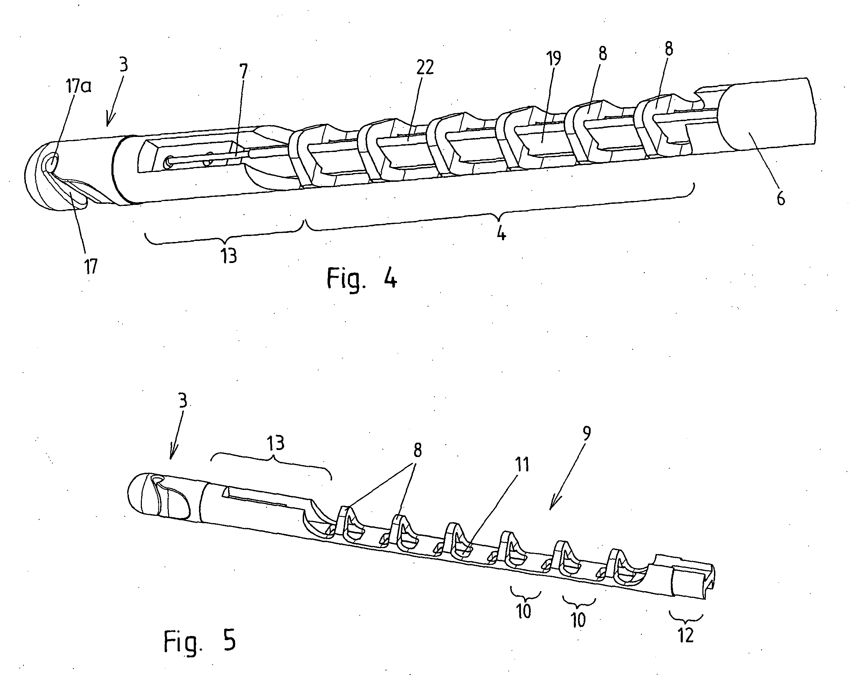

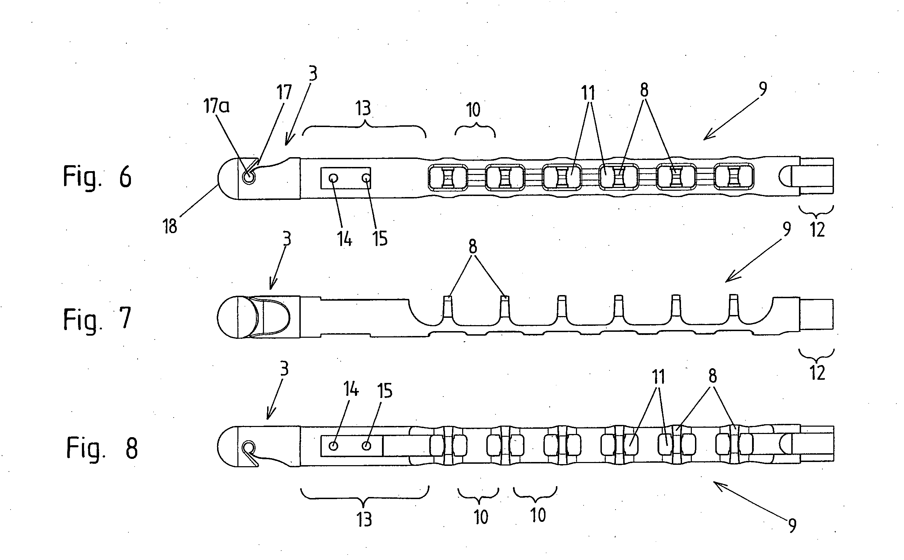

Über den krümmbaren Abschnitt 4 sind mehrere in Längsrichtung des krümmbaren Abschnitts 4 voneinander beabstandete Führungsbügel 8 angeordnet. Diese Führungsbügel 8 sind im gezeigten Ausführungsbeispiel Teile eines Formteils 9 (vgl. Fig. 5 bis 8). Aufeinanderfolgende Führungsbügel 8 sind jeweils durch einen Verbindungsabschnitt 10 des Formteils 9 miteinander verbunden. Die Führungsbügel 8 sind im Querschnitt des Schafts 2 (vgl. Fig. 3) U-förmig ausgebildet und weisen zwei Schenkel 8a, 8b auf, die über einen Bogenabschnitt miteinander verbunden sind. Die Verbindungsabschnitte 10 sind an den vom Bogenabschnitt abgelegenen Enden der Schenkel 8a, 8b angeformt. Es ist hierbei im Bereich eines Führungsbügels 8 jeweils eine Öffnung 11 in den Verbindungsabschnitten 10 freigelassen, wodurch die Biegbarkeit des Formteils 9 vergrößert wird.

An seinem proximalen Ende weist das Formteil 9 einen Einsteckabschnitt 12 zum Einstecken und Einkleben in das Rohr 6 auf. Distal des von den Führungsbügeln 8 und Verbindungsabschnitten 10 gebildeten Abschnitts (der mit dem krümmbaren Abschnitt 4 des Schafts 2 zusammenfällt) schließt ein Befestigungsabschnitt 13 an, in welchem das Zugelement 7 am Schaft 2 befestigt wird. Der Befestigungsabschnitt 13 ist hierfür ausreichend stabil ausgebildet und zur Befestigung des Zugelements 7 dienen die beiden Durchtrittsöffnungen 14, 15, durch welche das Zugelement 7 durchgeführt und hierbei umgebogen wird, wobei es in der weiteren Folge eingeklebt werden kann.

Distal schließt an den Befestigungsabschnitt 13 der Arbeitskopf 3 an, der in diesem Ausführungsbeispiel somit einstückig mit dem Formteil 9 ausgebildet ist. Der Arbeitskopf 3 stellt den über den (weiter unten beschriebenen) äußeren Hüllschlauch 24 vorstehenden Abschnitt des Formteils 9 dar. Der Arbeitskopf 3 ist mit einem von einer Seite des Arbeitskopfs 3 ausgehenden Schlitz 17 versehen, der sich zunächst verjüngt und anschließend an die Verjüngung einen vergrößerten Bereich 17a aufweist. Das stirnseitige bzw. distale Ende 18 des Arbeitskopfes 3 ist abgerundet ausgebildet. Mit dem Arbeitskopf 3 kann eine stumpfe Dissektion ausgeführt werden.

Über den krümmbaren Abschnitt 4 erstreckt sich weiters eine Blattfeder 19, welche im unbelasteten Zustand (d. h. ohne einwirkende äußere Kräfte) einen in Längsrichtung geraden Verlauf besitzt. Durch die flache, blattförmige Form wird eine hohe Biegesteifigkeit in lateraler Richtung erzielt, d. h. in eine Richtung senkrecht zur Ebene, in welcher der Schaft 2 in seinem gekrümmten Zustand liegt. Und zwar besteht diese laterale Biegesteifigkeit sowohl im gekrümmten als auch im geraden bzw. gestreckten Zustand des Schafts 2. Es kann dadurch eine sehr gute Kraftübertragung in dieser lateralen Richtung erzielt werden, wodurch beispielsweise eine stumpfe Dissektion erleichtert wird.

Die Biegesteifigkeit in Richtung der Krümmung (also in Richtung nach oben ausgehend vom gestreckten Zustand in Fig. 1) hängt von der Federkraft der Blattfeder 19 ab. Da durch das Betätigungsorgan 5 eine sehr große Kraftübersetzung erreichbar ist, kann die Blattfeder 19 entsprechend steif ausgelegt werden, sodass auch eine relativ hohe Biegesteifigkeit in dieser Richtung erzielt wird. In die entgegengesetzte Richtung (in Fig. 1 nach unten) ergibt sich eine hohe Biegesteifigkeit durch die Haltekraft des Zugelements 7.

Die Blattfeder 19 ist in achsialer Richtung des Schafts unverschiebbar angebracht.

Die Blattfeder 19 verläuft im gezeigten Ausführungsbeispiel zwischen den Schenkeln 8a, 8b der Führungsbügel, wobei sie im Bereich zwischen zwei Führungsbügeln 8 auf der den Führungsbügeln 8 zugewandten Seite der Verbindungsabschnitte 10 verläuft. Die Breite der Blattfeder 19 entspricht hierbei dem Abstand zwischen den Schenkeln 8a, 8b und die Blattfeder 19 ist gegen eine Abhebung von den Verbindungsabschnitten 10 durch Stufen der Schenkel 8a, 8b mit einem geringen Spiel gesichert.

Im distalen Endbereich ist die Blattfeder 19 an den Befestigungsabschnitt 13 angeklebt. Der proximale Endabschnitt verläuft unterhalb eines Steges 20 des Einsteckabschnitts 12 des Formteils 9 und steht über das Formteil 9 proximal etwas vor.

Ein jeweiliger Führungsbügel 8 begrenzt zusammen mit der Blattfeder 19 einen Kanal 21, durch den das Zugelement 7 verläuft.

Über die Länge des krümmbaren Abschnitts 4 ist das Zugelement 7 von einem biegbaren Gleitrohr 22 umgeben, das vorzugsweise von einem Teflonrohr gebildet wird, um die Gleitreibung des Zugelements 7 herabzusetzen.

Nachdem die Blattfeder 19 in das Formteil 9 eingesetzt und mit diesem verklebt worden ist und das Zugelement 7 in den Durchtrittsöffnungen 14, 15 befestigt werden und mit aufgeschobenem Gleitrohr 22 durch die Kanäle 21 verläuft, werden diese Teile mit einer Vergussmasse, vorzugsweise Silikon ausgegossen, wobei die Teile in eine entsprechende Form eingebracht werden. Es wird dadurch ein Teil mit einer glatten Außenkontur zwischen dem Arbeitskopf 3 und dem Einsteckabschnitt 12 ausgebildet, welche vorzugsweise zylindermantelförmig ist. Dieses Teil umfasst den krümmbaren Abschnitt 4 und erstreckt sich über die Länge des Formteils 9 mit Ausnahme des Arbeitskopfes 3 und des Einsteckabschnitts 12. Die Vergussmasse 23 ist der Übersichtlichkeit halber nur in Fig. 3 dargestellt.

In der Folge wird das ausgegossene Teil in das Rohr 6 eingeklebt. In der weiteren Folge wird ein Schrumpfschlauch über die Länge des Schaftes 2 aufgestülpt, der in der Folge aufgeschrumpft wird, wodurch sich ein äußerer Hüllschlauch 24 mit der aus den Fig. 1 bis 3 ersichtlichen Form ergibt. Dieser Hüllschlauch 24 besitzt zumindest im geraden Zustand des krümmbaren Abschnitts 4 über die gesamte Länge des Schafts 2 eine im Wesentlichen glatte äußere Oberfläche und schließt den Schaft 2 gegen ein Eindringen von Flüssigkeiten ab. Durch diese hermetisch geschlossene Ausbildung des Schafts 2 wird eine rückstandsfreie Reinigung des Instruments nach einer Operation ermöglicht. Durch die im Wesentlichen glatte Ausbildung der äußeren Oberfläche des Hüllschlauchs 24 wird diese Reinigung noch erleichtert.

Zur Fixierung einer eingestellten Schwenkposition des Betätigungsorgans 5 und somit einer eingestellten Krümmung des krümmbaren Abschnitts 4 ist eine mit dem Betätigungsorgan 5 zusammenwirkende Rasteinrichtung vorhanden. Diese umfasst ein verschwenkbares Rastteil 25, das mit einer mit dem Betätigungsorgan 5 zusammenwirkenden Verzahnung versehen ist. Durch eine Feder 26 wird die Verzahnung gegen ein Rastelement des Betätigungsorgans 5 gedrückt. Bei einer Verschwenkung des Betätigungsorgans 5 in Richtung des Pfeils 27 rastet das Rastelement in den entsprechenden Zahn der Verzahnung des Rastteils 25 ein. Die Verrastung kann durch eine manuelle Verschwenkung des Rastteils 25 in Richtung des Pfeils 28 wiederum gelöst werden.

Bei einer Verschwenkung des Betätigungsorgans 5 in Richtung des Pfeils 27 wird das Zugelement 7 in Richtung des Pfeils 29 verschoben, wobei der krümmbare Abschnitt 4 unter Überwindung der Rückstellkraft der Blattfeder 19 zunehmend gekrümmt wird. Die Krümmung des krümmbaren Abschnitts 4 ist hierbei zwischen der geraden Stellung und der maximal gekrümmten Stellung stufenlos verstellbar. In der maximal gekrümmten Stellung kann bei einem erfindungsgemäßen Instrument ein Winkel 30 der Verschwenkung des Arbeitskopfs 3 um mehr als 90° erreicht werden, wobei in der Praxis Winkel von über 110° erreichbar sind.

Im gekrümmten Zustand des krümmbaren Abschnitts wirkt auf das Zugelement eine Kraft in Richtung einer Abhebung des Zugelements von der Blattfeder 19. Durch die Führungsbügel 8 wird eine solche Abhebung des Zugelements 7 gegenüber der Blattfeder 19 begrenzt. Im gezeigten Ausführungsbeispiel weist das Zugelement von der Blattfeder 19 im geraden und im gekrümmten Zustand des krümmbaren Abschnitts im Wesentlichen den gleichen Abstand auf.

Das Formteil 9 besteht aus einem Kunststoffmaterial, welches einerseits eine ausreichende Flexibilität aufweist, um ohne Beschädigung gebogen werden zu können, und andererseits eine ausreichende Festigkeit besitzt, um den vom Zugelement 7 ausgeübten Kräften Stand zu halten, beispielsweise aus Polysulfon. Der Hüllschlauch 24 kann beispielsweise von einem PTFE-Schrumpfschlauch gebildet werden. Anstelle von Teflon kann auch ein anderes medizinisch zugelassenes weiches Kunststoffmaterial eingesetzt werden. Die Blattfeder 19 kann beispielsweise aus Edelstahl bestehen. Das Zugelement 7 kann beispielsweise von einem Metalldraht oder Metallseil gebildet werden.

Mit einem erfindungsgemäßen Instrument kann in vorteilhafter Weise eine stumpfe Dissektion durchgeführt werden, wobei Gewebelagen voneinander separiert werden. Mit dem Arbeitskopf 3 können hierbei Strukturen im Körperinneren umfahren werden. Beispielsweise Hohlorgane oder Gefäße, zu welchem Zweck die Krümmung bzw. Biegung des krümmbaren Abschnitts 4 entsprechend eingestellt wird. Mit dem Arbeitskopf 3 können weiters beispielsweise Gegenstände, wie Fäden oder Implantate, um Hohlorgane oder Gefäße herumgezogen werden. Beispielsweise kann ein erfindungsgemäßes Instrument bei der Transplantation eines Magenbandes vorteilhaft eingesetzt werden.

Unterschiedliche Modifikationen des beschriebenen Ausführungsbeispiels der Erfindung sind denkbar und möglich, ohne den Bereich der Erfindung zu verlassen. So wäre es beispielsweise denkbar und möglich, die Führungsbügel direkt an der Blattfeder 19 festzulegen, wobei ein sich über die Länge des krümmbaren Abschnitts 4 erstreckendes Formteil auch entfallen könnte. Beispielsweise könnten die Führungsbügel 8 an der Blattfeder 19 mit den beiden Enden ihrer Schenkel angeschweißt sein. Die Führungsbügel 8 könnten hierbei z. B. von einem gebogenen Metalldraht gebildet werden.

Weiters wäre es auch denkbar und möglich, die Vergussmasse aus Silikon wegzulassen, wenn ein Hüllschlauch aus einem ausreichend stabilen Material eingesetzt wird, damit sich nicht zu starke Unebenheiten an der Außenseite des Hüllschlauchs ergeben, welche den Operationseinsatz des Instruments beeinträchtigen. Der Hüllschlauch könnte auch in anderer Weise als durch einen Schrumpfschlauch ausgebildet sein. Denkbar und möglich wäre es auch, den Hüllschlauch wegzulassen und nur eine Vergussmasse vorzusehen, die die äußere Oberfläche des Schaftes im krümmbaren Abschnitt bildet, dies insbesondere dann, wenn das Instrument nur als Einmalinstrument zum Einsatz kommt.

Die Blattfeder 19 könnte beispielsweise auch aus einer formspeichernden Legierung (einem Material mit sogenanntem Formgedächtnis) bestehen. Die Rückstelleigenschaften von solchen formspeichernden Legierungen werden auch als "pseudoelastisch" bezeichnet. Beispielsweise könnten hier Nickel-Titan-Legierungen zum Einsatz kommen.

Auch andere Betätigungsorgane als verschwenkbare Handgriffe sind denkbar und möglich. So könnte beispielsweise eine drehbar gelagerte Mutter vorhanden sein, in deren Gewinde eine gegenüber einer Verdrehung gesicherte Gewindestange ragt, an der das Zugelement 7 befestigt ist. Prinzipiell könnte auch mehr als ein einzelnes Zugelement vorhanden sein. Die Führungsbügel 8 könnten auch im Bereich zwischen dem Zugelement 7 und der Blattfeder 19 geschlossen ausgebildet sein, wodurch getrennte Kanäle zur Durchführung des Zugelements 7 und der Blattfeder 19 ausgebildet werden könnten.

### Legende zu den Hinweisziffern:

- 1: Handgriff
- 2: Schaft
- 3: Arbeitskopf
- 4: krümmbarer Abschnitt
- 5: Betätigungsorgan
- 6: Rohr
- 7: Zugelement
- 8: Führungsbügel
- 8a: Schenkel
- 8b: Schenkel
- 9: Formteil
- 10: Verbindungsabschnitt
- 11: Öffnung
- 12: Einsteckabschnitt
- 13: Befestigungsabschnitt
- 14: Durchtrittsöffnung
- 15: Durchtrittsöffnung
- 17: Schlitz
- 17a: vergrößerter Bereich
- 18: distales Ende
- 19: Blattfeder
- 20: Steg
- 21: Kanal
- 22: Gleitrohr
- 23: Vergussmasse
- 24: Hüllschlauch
- 25: Rastteil
- 26: Feder
- 27: Pfeil
- 28: Pfeil
- 29: Pfeil
- 30: Winkel

## Patentansprüche

1. Laparoskopisches Instrument mit einem Handgriff (1), einem vom Handgriff (1) getragenen Schaft (2) und einem am distalen Ende des Schafts (2) angeordneten Arbeitskopf (3), wobei der Schaft (2) einen distalen, krümmbaren Abschnitt (4) aufweist und zur Verstellung der Krümmung des krümmbaren Abschnitts (4) ein von einem Draht oder Seil gebildetes Zugelement (7) vom Bereich des Handgriffs (1) bis über den krümmbaren Abschnitt (4) verläuft und auf der distalen Seite des krümmbaren Abschnitts (4) am Schaft (2) oder am Arbeitskopf (3) befestigt ist und ein im Bereich des Handgriffs (1) angeordnetes Betätigungsorgan (5) mit dem Zugelement (7) zur Zugbeaufschlagung des Zugelements (7) zusammenwirkt und wobei über den krümmbaren Abschnitt (4) mehrere in Längsrichtung des krümmbaren Abschnitts (4) voneinander beabstandete Führungsbügel (8) für das Zugelement (7) angeordnet sind, **dadurch gekennzeichnet, dass** sich über den krümmbaren Abschnitt (4) weiters eine im unbelasteten Zustand gerade Blattfeder (19) erstreckt, wobei die Abhebung des Zugelements (7) von der Blattfeder (19) durch die Führungsbügel (8) begrenzt ist.

2. Laparoskopisches Instrument nach Anspruch 1, **dadurch gekennzeichnet, dass** ein jeweiliger Führungsbügel (8) zusammen mit dem Federelement (19) einen Kanal (21) begrenzt, durch den sich das Zugelement (7) erstreckt.

3. Laparoskopisches Element nach Anspruch 1 oder Anspruch 2, **dadurch gekennzeichnet, dass** ein jeweiliger Führungsbügel (8) im Querschnitt des Schafts (2) U-förmig ausgebildet ist.

4. Laparoskopisches Element nach einem der Ansprüche 1 bis 3, **dadurch gekennzeichnet, dass** ein Formteil (9) aus Kunststoff vorhanden ist, welches sich über den krümmbaren Abschnitt (4) erstreckt und die Führungsbügel (8) aufweist.

5. Laparoskopisches Instrument nach Anspruch 4, **dadurch gekennzeichnet, dass** aufeinanderfolgende Führungsbügel (8) jeweils durch einen Verbindungsabschnitt (10) des Formteils (9) verbunden sind, wobei die Verbindungsabschnitte (10) vorzugsweise an den Enden der Schenkel (8a, 8b) der U-förmigen Führungsbügel (8) angeformt sind.

6. Laparoskopisches Instrument nach Anspruch 4 oder Anspruch 5, **dadurch gekennzeichnet, dass** das Formteil (9) auch den Arbeitskopf (3) bildet.

7. Laparoskopisches Element nach einem der Ansprüche 4 bis 6, **dadurch gekennzeichnet, dass** die Blattfeder (19) am Formteil (9) distal und/oder proximal des krümmbaren Abschnitts (4) befestigt ist, vorzugsweise angeklebt ist.

8. Laparoskopisches Element nach einem der Ansprüche 4 bis 7, **dadurch gekennzeichnet, dass** das Formteil (9) am distalen Ende eines mit seinem proximalen Ende am Handgriff (1) befestigten Rohrs (6) befestigt ist.

9. Laparoskopisches Element nach einem der Ansprüche 1 bis 8, **dadurch gekennzeichnet, dass** das Zugelement (7) zumindest über die Länge des krümmbaren Abschnitts (4) von einem biegbaren Gleitrohr (22) umgeben ist.

10. Laparoskopisches Instrument nach einem der Ansprüche 1 bis 9, **dadurch gekennzeichnet, dass** zumindest der krümmbare Abschnitt (4) mit einer Vergussmasse (23), vorzugsweise Silikon, auf eine zumindest über die Länge des krümmbaren Abschnitts (4) glatte Außenkontur ausgegossen ist, die vorzugsweise zylindermantelförmig ist.

11. Laparoskopisches Instrument nach einem der Ansprüche 1 bis 10, **dadurch gekennzeichnet, dass** der krümmbare Abschnitt (4) von einem äußeren Hüllschlauch (24) umgeben ist, der sich vorzugsweise über die gesamte Länge des Schafts (2) erstreckt.

12. Laparoskopisches Instrument nach einem der Ansprüche 1 bis 11, **dadurch gekennzeichnet, dass** das Betätigungsorgan (5) von einem verschwenkbar am Handgriff (1) gelagerten Betätigungsgriff gebildet wird, an dem das Zugelement (7) befestigt ist.

13. Laparoskopisches Instrument nach Anspruch 12, **dadurch gekennzeichnet, dass** eine lösbare Rasteinrichtung mit einem mit dem Betätigungsorgan (5) zusammenwirkenden Rastteil (25) zur Fixierung einer eingestellten Schwenkposition des Betätigungsorgans (5) vorhanden ist.

14. Laparoskopisches Instrument nach einem der Ansprüche 1 bis 13, **dadurch gekennzeichnet, dass** der Arbeitskopf (3) in der am stärksten gekrümmten Stellung des krümmbaren Abschnitts (4) gegenüber der geraden Ausgangslage des krümmbaren Abschnitts (4) um einen Winkel (30) von mehr als 90° verschwenkt ist.
